# EUROPÄISCHE PATENTSCHRIFT

(11) **EP 0 325 559 B1**
(45) Veröffentlichungstag und Bekanntmachung des Hinweises auf die Patenterteilung: **15.12.1993**
(21) Anmeldenummer: 89810018.5
(22) Anmeldetag: 11.01.1989
(51) Int. Cl.: C07C 259/06, C07D 309/40, C07D 213/68, C07F 15/02, C07D 417/04

(54) **Verfahren zur Herstellung von Komplexverbindungen**
Process for the preparation of complex compounds
Procédé pour la préparation de composés complexes

(30) Priorität: 20.01.1988 CH 184/88
(43) Veröffentlichungstag der Anmeldung: 26.07.1989
(73) Patentinhaber: CIBA-GEIGY AG, 4002 Basel (CH)
(72) Erfinder: Peter, Heinrich H., Dr., CH-4102 Binningen (CH); Moerker, Théophile, CH-4414 Füllinsdorf (CH)

(56) Entgegenhaltungen:
- EP-A- 0 159 194
- EP-A- 0 235 361
- HELVETICA CHIMICA ACTA, vol. 46, no. 4, 1963, Seiten 1409-1422; G. ANDEREGG et al.: "Hydroxamatkomplexe III). Eisen(III)-Austausch zwischen Sideraminen und Komplexonen. Diskussion der Bildungskonstanten der Hydroxamatkomplexe"
- "The Merck Index2, 11th Ed.. (1989), Seite 1452

## Beschreibung

Die Erfindung betrifft ein neuartiges Verfahren (Methodikverfahren) zur Herstellung von Metallkomplexen, insbesondere von paramagnetischen und/oder radioaktiven Chelatkomplexen, in besonders reiner Form, die nach diesen Verfahren hergestellten Komplexe sowie neue Chelatkomplexe mit bekannten Chelatbildnern.

Paramagnetische und/oder radioaktive Chelatkomplexe werden hauptsächlich in der diagnostischen Medizin, z.B. der Röntgen-, Radionuklid-, Ultraschall- und/oder magnetischen Kernresonanzdiagnostik, als Kontrastmittel verwendet. Für diese Verwendung ist es wesentlich, die Chelatkomplexe in grösstmöglicher Reinheit herzustellen. Bei den bisher bekannten Verfahren zur Herstellung der Chelatkomplexe wird eine anorganische Metallverbindung, meist ein Halogenid, wie Chlorid, mit dem Chelatbildner umgesetzt. Die so hergestellten Komplexe weisen jedoch nicht die gewünschte Reinheit auf. Vielmehr sind sie verunreinigt durch das in der anorganischen Metallverbindung enthaltene Gegenion, im Ueberschuss eingesetztes oder unumgesetztes Edukt sowie durch Produkte, die beim Neutralisieren von Säure, wie Halogenwasserstoffsäure oder Schwefelsäure, gebildet werden, welche während der Chelatbildung entsteht. Die Neutralisation ist erforderlich, weil die für medizinische Zwecke verwendeten Chelatkomplexe einen physiologisch verträglichen pH-Wert aufweisen müssen. Die Verunreinigungen lassen sich nur schwer und unvollständig abtrennen.

Der Erfindung liegt die Aufgabe zugrunde, ein einfaches Verfahren zur Herstellung von Chelatkomplexen in reinerer Form zu entwickeln, welches ohne Neutralisationsschritt auskommt.

Insbesondere betrifft die Erfindung ein Verfahren zur Herstellung eines Komplexes aus einem Metallion und einem Chelatbildner, dadurch gekennzeichnet, dass man einen Komplex aus einer β-Dicarbonylverbindung und dem genannten Metallion, welcher in einem mit Wasser nicht beliebig mischbaren organischen Lösungsmittel gut löslich ist, mit einer stöchiometrischen Menge oder einem Unterschuss eines Chelatbildners, dessen Bindungsaffinität zum Metallion höher als diejenige der β-Dicarbonylverbindung ist, oder eines Salzes, vorzugsweise eines pharmazeutisch verwendbaren Salzes, eines solchen Chelatbildners mit mindestens einer salzbildenden Gruppe umkomplexiert.

Die zu komplexierenden Metallionen sind insbesondere paramagnetische Metallionen aus der Reihe der Uebergangsmetalle einschliesslich der Lanthaniden und Actiniden, sowie Metallionen aus der dritten Hauptgruppe des Periodensystems und Radionuklidionen.

Aus der Reihe der paramagnetischen Uebergangsmetallionen ausschliesslich der Lanthaniden und Actiniden müssen an erster Stelle die Eisenionen Fe^{2⁺} und vor allem Fe^{³⁺} erwähnt werden, weiter das Kupferion Cu^{²⁺}, das Cobaltion Co^{²⁺}, das Nickelion Ni^{²⁺}, die Manganionen Mn^{²⁺} und Mn^{³⁺}, die Chromionen Cr^{²⁺} und Cr^{³⁺} sowie das Vanadinion V^{²⁺}.

Aus der Reihe der Lanthanidionen sei besonders das Gadoliniumion Gd^{³⁺} hervorgehoben, aber auch das Europiumion Eu^{²⁺}, das Lanthanion La^{³⁺} und das Ytterbiumion Yb^{³⁺} sind zu nennen.

Aus der Reihe der Actiniden sei das Protactiniumion Pa^{⁴⁺} hervorgehoben.

Metallionen aus der dritten Hauptgruppe des Periodensystems sind Aluminium- sowie vorzugsweise Gallium- und Indiumionen, wobei bei Gallium und Indium hauptsächlich die radioaktiven Isotope, z.B. ⁶⁷Ga und ¹¹¹In im Vordergrund stehen.

Radionuklidionen sind insbesondere die Ionen der radioaktiven Isotope der obengenannten Metalle, z.B. des metastabilen Technetium-99, ^{99m}Tc, oder ¹⁴⁰La, ¹⁶⁸Yb, ⁶⁷Ga oder ¹¹¹In.

Chelatbildner sind organische Verbindungen, die mindestens zwei potentielle Liganden aufweisen, wie insbesondere die Desferrioxamine mit freien OH-Gruppen, die z.B. aus dem US-Patent 3 634 407 bekannt sind, vorzugsweise die Desferrioxamine der B-Reihe, hauptsächlich das als Methansulfonat unter der Bezeichnung Desferal® käufliche Desferrioxamin B oder Derivate davon mit acylierter Aminogruppe, aber auch Desferrioxamin E. Weitere bevorzugte Chelatbildner, insbesondere für Fe³⁺, Al³⁺ und Cr³⁺, sind z.B. die aus dem Europäischen Patent Nr. 45 281 bekannte 2-(3'-Hydroxy-pyrid-2'-yl)-4-methyl-2-thiazolin-4-carbonsäure, die nachstehend als Desferrithiocin bezeichnet wird, und dessen dort beschriebenes Desmethyl-Derivat, sowie weitere von Mikroorganismen gebildete Siderophore, z.B. Rhodotorulasäure.

Zahlreiche weitere Chelatbildner, z.B. 3-Hydroxy-2-methyl-4H-pyran-4-on (Maltol), (L)-2-Amino-3-[3-hydroxy-pyrid-4-on-1-yl]-propionsäure (L-Mimosin) und andere 3-Hydroxy-4-pyridonderivate, kommen in Frage, wobei die konkrete Auswahl durch die gewünschten Eigenschaften des herzustellenden Chelatkomplexes bestimmt wird (siehe unten).

Salzbildende Gruppen in einem Chelatbildner sind saure Gruppen, z.B. Carboxyl-, Phosphorsäure- oder Sulfonsäuregruppen, oder basische Gruppen, z.B. Aminogruppen.

Salze von Chelatbildnern, die, wie Desferrithiocin, mindestens eine saure Gruppe aufweisen, sind vorzugsweise Alkalimetallsalze, hauptsächlich Natrium- oder Kaliumsalze. Salze von Chelatbildnern, die, wie Desferrioxamin B, mindestens eine basische Gruppe aufweisen, sind Säureadditionssalze, vorzugsweise pharmazeutisch verwendbare Säureadditionssalze, z.B. mit anorganischen Säuren, wie Salz-, Schwefel- oder Phosphorsäure, oder mit geeigneten organischen Carbon- oder Sulfonsäuren, z.B. Trifluoressig-säure oder Methylsulfonsäure.

Eine β-Dicarbonylverbindung ist eine organische Verbindung, die zwei Carbonylgruppen in 1,3-Stellung zueinander enthält und auch in der Enolform vorliegen kann, wobei die zwei Carbonylgruppen für die Komplexierung eines Metallions verfügbar sein müssen und nicht sterisch gehindert sein dürfen. Eine bevorzugte 1,3-Dicarbonylverbindung ist 2,4-Pentan-dion (Acetylaceton), weil die Acetylacetonate zahlreicher Metalle im Handel erhältlich sind.

Ein mit Wasser nicht beliebig mischbares organisches Lösungsmittel ist z.B. ein entsprechender Carbonsäureester, wie Essigsäureethylester, ein entsprechender cyclischer oder vornehmlich acyclischer Ether, wie Diethylether, oder ein unsubstituierter oder halogenierter Kohlenwasserstoff, z.B. ein aromatischer Kohlenwasserstoff, wie Benzol oder Toluol, ein aliphatischer Kohlenwasserstoff, wie Pentan oder Heptan, oder ein halogenierter Kohlenwasserstoff, wie Chloroform oder Dichlormethan.

Welche von den vorstehend genannten Lösungsmitteln einen bestimmten Metallkomplex, enthaltend eine β-Dicarbonylverbindung, gut lösen, hängt von dem speziellen Komplex ab. Metallacetylacetonate zum Beispiel sind in Essigsäureethylester, Diethylether, Benzol oder Toluol gut löslich.

Die Bindungsaffinität des Chelatbildners zum Metallion muss genügend höher sein als die Bindungsaffinität der β-Dicarbonylverbindung zum entsprechenden Metallion, d.h. der negative dekadische Logarithmus der Dissoziationskonstanten (pK) muss für den Komplex aus Chelatbildner und Metallion grösser sein als für den Komplex aus β-Dicarbonylverbindung und Metallion, weil das erfindungsgemässe Verfahren sonst nicht oder nicht quantitativ verläuft.

Die Metallkomplexe eines Desferrioxamins werden nachfolgend in Analogie zur gebräuchlichen Bezeichnung des Eisen(III)-komplexes als "Ferrioxamin" unter Angabe des komplexierten Metalls und, wenn nötig, dessen Oxidationsstufe, gefolgt von dem Suffix "oxamin" bezeichnet. Analog werden die von Desferrithiocin gebildeten Komplexe unter Verwendung des Suffixes "thiocin" benannt.

Wenn der nach dem erfindungsgemässen Verfahren hergestellte Chelatkomplex in der medizinischen Diagnostik verwendet werden soll, muss er unter anderem die folgenden Eigenschaften haben:
Insbesondere dann, wenn das Metallion in freier Form giftig ist, muss der Komplex möglichst stabil sein, damit möglichst wenig freie Metallionen in den Organismus gelangen. Wenn das betreffende Metallion körpereigen und in der jeweiligen Konzentration ungiftig ist, kann eine weniger hohe Stabilität des Komplexes in Kauf genommen werden. Als körpereigene Ionen werden Eisenionen für das erfindungsgemässe Verfahren bevorzugt verwendet. Selbstverständlich sollte auch der Chelatkomplex als Ganzes möglichst wenig giftig und für die meisten Anwendungen genügend löslich sein sowie möglichst bald nach Durchführung der Diagnose aus dem Organismus ausgeschieden werden. Die obengenannten Anforderungen werden z.B. durch die Eisen(III)-komplexe von Desferrioxamin B und Desferrithiocin in hervorragender Weise erfüllt.

Zur Durchführung des Verfahrens wird eine Lösung des Komplexes aus der β-Dicarbonylverbindung und dem Metallion in einem geeigneten Lösungsmittel, in welchem er gut löslich ist, vorzugsweise einem mit Wasser nicht oder nur wenig mischbaren organischen Lösungsmittel, z.B. einem geeigneten Ester, wie Essigsäureethylester, oder einem geeigneten Ether, wie Diethylether, zu einer Lösung des Chelatbildners in einem geeigneten Lösungsmittel gegeben und intensiv gerührt. Wenn es die Löslichkeit des Chelatbildners erlaubt, z.B. im Falle von Desferrioxamin B-mesylat, ist das Lösungsmittel für den Chelatbildner vorteilhafterweise Wasser. Im Falle von nur wenig wasserlöslichen Chelatbildnern, kann auch eine Suspension des Chelatbildners in Wasser verwendet werden. Der Chelatbildner kann aber auch in einem nicht-wässerigen Lösungsmittel, z.B. einem Alkohol, wie Methanol, Ethanol oder Isopropanol, eingesetzt werden. Die Reaktanden können in äquivalenten Mengen eingesetzt werden. Man kann aber auch einen leichten Ueberschuss, z.B. 10-20 % des Komplexes mit der β-Dicarbonylverbindung verwenden. Die Reaktion wird vorzugsweise bei einer Temperatur zwischen etwa -20°C und etwa +150°C, insbesondere zwischen 0°C und +100°C, vornehmlich zwischen +10°C und +70°C, in erster Linie zwischen +15°C und +40°C, hauptsächlich bei Raumtemperatur (etwa +20°C) durchgeführt. Die im Einzelfall anzuwendende Reaktionstemperatur hängt unter anderem von den Schmelz- und Siedepunkten des Lösungsmittel(gemische)s, von der Stabilität der Reaktanden und des Chelatkomplexes und der gewünschten Reaktionsgeschwindigkeit ab. Wenn erwünscht oder erforderlich, kann die Reaktion unter Druck, z.B. unter dem Eigendruck des Systems, und/oder in einer Inertgasatmosphäre, z.B. unter Stickstoff oder Argon, durchgeführt werden. Die isolierten Reinausbeuten betragen etwa 80-100 % der Theorie.

Zur Isolierung des gewünschten Metallkomplexes und zur Abtrennung von unumgesetztem Edukt und gebildetem Nebenprodukt, d.h. von dem Komplex aus der β-Dicarbonylverbindung und dem Metallion sowie von der freigesetzten β-Dicarbonylverbindung, macht man sich Unterschiede in der relativen Löslichkeit zwischen dem gewünschten Metallkomplex und dem Edukt sowie Nebenprodukt zunutze. Dabei wird zweckmässigerweise bereits für die Reaktion ein Lösungsmittelsystem gewählt, dass für die leichte Isolierung des gewünschten Metallkomplexes geeignet ist.

Die Komplexe mit der β-Dicarbonylverbindung, z.B. die Metall-acetylacetonate, sind in Wasser unlöslich, in einem mit Wasser praktisch nicht mischbaren Lösungsmittel, wie Essigsäureethylester oder Diethylether, dagegen löslich. Demgegenüber sind die erfindungsgemäss gebildeten Komplexe, z.B. die Desferrioxamin B-Chelatkomplexe, in mindestens einem mit Wasser praktisch nicht mischbaren organischen Lösungsmittel, z.B. Essigsäureethylester, Diethylether, Benzol oder Toluol, praktisch unlöslich, was deren leichte Abtrennung und Reinigung erlaubt. Die Desferrithiocin-Komplexe werden vorzugsweise in einem System aus Wasser und einem weniger polaren Lösungsmittel als Essigsäureethylester, z.B. in Diethylether, hergestellt.

Nach beendeter Umkomplexierung wird in dem Regelfall, in dem das Reaktionsgemisch Wasser enthält, die wässerige Phase abgetrennt und mit einem organischen Lösungsmittel extrahiert, in dem der gewünschte Metallkomplex möglichst schlecht und die Verunreinigungen möglichst gut löslich sind. Anschliessend wird die wässerige Phase, erforderlichenfalls nachdem sie zuvor eingeengt wurde, lyophilisiert. In dem Ausnahmefall, in dem das Reaktionsgemisch kein Wasser enthält, wird das Reaktionsgemisch stark eingeengt, z.B. bis zur Trockne, und dann mit einem organischen Lösungsmittel extrahiert, in dem der gewünschte Metallkomplex möglichst schlecht und die Verunreinigungen möglichst gut löslich sind.

Die Komplexe mit der β-Dicarbonylverbindung sind im Handel erhältlich, z.B. im Falle zahlreicher Acetylacetonate, oder können in an sich bekannter Weise, z.B. durch Umsetzung der β-Dicarbonylverbindung mit einem Salz des entsprechenden Metalls, z.B. einem Chlorid, hergestellt werden. Man kann auch Metallsalze der 2-Ethylcapronsäure (Octoate), Metallnaphthenate oder -stearate mit der β-Dicarbonylverbindung umsetzen [G. Stöckelmann et al., Angew. Chem. 79, 530 (1967)] oder mit dem gewünschten Metallion beladene Kationenaustauscher in einem organischen Lösungsmittel mit der β-Dicarbonylverbindung in Kontakt bringen [K. Ohzeki et al., Bull. Chem. Soc. Jap. 48, 67-68 (1975)].

Eine bevorzugte Ausführungsform des erfindungsgemässen Verfahrens ist dadurch gekennzeichnet, dass man ein Acetylacetonat eines Radionuklidions oder eines paramagnetischen Metallions aus der Reihe der Uebergangsmetalle einschliesslich der Lanthaniden, vorzugsweise ein Acetylacetonat von Fe^{²⁺}, Fe^{³⁺}, Cu^{²⁺}, Co^{²⁺}, Ni^{²⁺}, Mn^{²⁺}, Mn^{³⁺}, Cr^{²⁺}, Cr^{³⁺}, V^{²⁺}, Gd^{³⁺}, Eu^{²⁺}, La^{³⁺} oder Yb^{³⁺}, mit einem Chelatbildner, ausgewählt aus Desferrioxamin B, Desferrioxamin E und Desferrithiocin und einem pharmazeutisch verwendbaren Salz davon, umkomplexiert.

Eine weitere bevorzugte Ausführungsform des erfindungsgemässen Verfahrens ist dadurch gekennzeichnet, dass man ein Acetylacetonat von Eisen(III), Mangan(III), Indium(III) oder Gallium(III) mit Desferrioxamin B, Desferrioxamin E, Desferrithiocin, Maltol, L-Mimosin, 3-Hydroxy-1,2-dimethyl-4-pyridon, 3-Hydroxy-2-methyl-N-propyl-4-pyridon oder Rhodotorulasäure in Wasser-Essigsäureethylester oder Wasser-Diethylether bei Raumtemperatur umsetzt.

Die Erfindung betrifft auch die nach dem erfindungsgemässen Verfahren hergestellten Chelatkomplexe, neue, d.h. nicht zum Stand der Technik gehörende Chelatkomplexe, insbesondere die in den Beispielen beschriebenen Chelatkomplexe, soweit diese neu sind, und die Verwendung dieser Chelatkomplexe in der diagnostischen Medizin.

Die radioaktive Metallionen, z.B. ^{99m}Tc, ¹¹¹In, ⁶⁷Ga, ¹⁴⁰La oder ¹⁶⁸Yb, enthaltenden Chelatkomplexe können z.B. als Radiopharmazeutika verwendet werden. Chelatkomplexe mit stabilen Isotopen, die ein höheres Atomgewicht als Iod haben absorbieren Röntgenstrahlen und können daher als Röntgenkontrastmittel verwendet werden. Einige der letztgenannten Chelatkomplexe absorbieren, reflektieren oder streuen Ultraschallwellen und können daher auch in der Ultraschalldiagnose eingesetzt werden. Chelatkomplexe, die ein paramagnetisches Metallion, z.B. Gd^{³⁺}, Mn^{²⁺}, Cr^{³⁺} oder Fe^{³⁺}, mit symmetrischem elektronischen Grundzustand enthalten, beschleunigen die Spinrelaxation und können in der NMR-Spektroskopie als Kontrastmittel verwendet werden. Chelatkomplexe, die ein paramagnetisches Metallion mit unsymmetrischem elektronischen Grundzustand enthalten, können in der NMR-Spektroskopie oder in der magnetischen in vivo Resonanzspektroskopie als Verschiebungsreagenz verwendet werden. Aluminiumkomplexe können als Vergleichsverbindungen bei der Evaluierung (z.B. Toxizitätsprüfung) von Chelatbildnern verwendet werden.

Die einem Säugetier zu verabreichende Dosis hängt unter anderem vom Chelatkomplex, der Art des Säugetieres und dem Verwendungszweck ab und liegt z.B. in der Grössenordnung von 0,001-1 Millimol/Kilogramm Körpergewicht. Die Verabreichung erfolgt vorzugsweise parenteral, insbesondere intravenös, oder enteral, z.B. oral.

Die nachfolgenden Beispiele illustrieren die Erfindung, ohne sie einzuschränken.

### Abkürzungen

- DMSO:: Dimethylsulfoxid
- FAB:: Fast Atom Bombardment
- HPLC:: Hochdruckflüssigkeitschromatographie

Beispiel 1: Eine Lösung von 2,20 kg (5,66 Mol) käuflichen Eisen(III)-acetylacetonats in 25 Liter Essigsäureethylester wird unter intensivem Rühren bei Raumtemperatur mit einer Lösung von 3,38 kg (5,15 Mol) Desferrioxamin B-methansulfonat in 20 Liter Wasser versetzt und 1 Stunde intensiv gerührt, worauf sofort eine Rotfärbung erscheint. Die wässerige Phase wird viermal mit je 10 Liter Essigsäureethylester extrahiert, zur Entfernung des restlichen Essigesters bei 55°C und 85'000 Pa (0,85 bar) etwas eingeengt und anschliessend lyophilisiert. Das Lyophilisat wird mit Essigsäureethylester digeriert und im Hochvakuum getrocknet. Man erhält 3,54 kg (98 % der Theorie) tiefrotes, hygroskopisches Ferrioxamin B-methansulfonat, das 1 Mol Wasser enthält.

| C₂₅H₄₅FeN₆O₈·CH₃SO₃H·H₂O (727,633) | | | | | |
|---|---|---|---|---|---|
| Ber. | C 42,92 | H 7,06 | Fe 7,68 | N 11,55 | S 4,41 |
| Gef. | C 43,15 | H 7,19 | Fe 7,81 | N 11,60 | S 4,44 |

### HPLC:

Säule: Hypersil ODS, 5 µm, 120 x 4,6 mm
Systeme: Lösung A = 2,5 mMol Phosphatpuffer pH 3,0
Lösung B = 20 % Lösung A und 80 % Acetonitril
Gradient:

| Minuten | % A | % B | Fluss: ml/min |
|---|---|---|---|
| 0 | 100 | 0 | 2,3 |
| 10 | 70 | 30 | 2,3 |
| 12 | 0 | 100 | 2,3 |
| 15 | 100 | 0 | 2,3 |

R_{f}-Wert: 7 Minuten,
Massenspektrum [(+) FAB in Thioglycerin]: (M+H)⁺ = 614.
Löslichkeiten: gut löslich in Wasser.

Beispiel 2: 26,40 g (40 mMol) Desferrioxamin B-methansulfonat in 400 ml Wasser werden unter intensivem Rühren mit 16,80 g (48 mMol) Mangan(III)-acetylacetonat versetzt und 2 Stunden bei Raumtemperatur intensiv gerührt. Nach Aufarbeitung analog Beispiel 1 erhält man tiefgrünes, schwach hygroskopisches Mangan(III)-oxamin B-methansulfonat, das 0,5 Mol Wasser enthält.

| C₂₅H₄₅MnN₆O₈·CH₃SO₃H·1/2 H₂O (717,708) | | | | | |
|---|---|---|---|---|---|
| Ber. | C 43,51 | H 7,02 | N 11,71 | S 4,46 | Mn 7,66 |
| Gef. | C 43,38 | H 7,02 | N 11,50 | S 4,29 | Mn 8,16 |

- HPLC (Bedingungen wie in Beispiel 1):: R_{f}-Wert = 5,5 Minuten,
- Löslichkeiten:: gut löslich in Wasser.

Beispiel 3: Analog Beispiel 2, jedoch nach nur einstündigem Rühren und Digerieren des Lyophilisats mit Diethylether/n-Heptan, erhält man aus 13,12 g (20 mMol) Desferrioxamin B-methansulfonat in 250 ml Wasser und 7,78 g (24 mMol) Aluminium-acetylacetonat in 200 ml Essigsäureethylester weisses, hygroskopisches Aluminiumoxamin B-methansulfonat, das 1,5 Mol Wasser enthält.

| C₂₅H₄₅AlN₆O₈·CH₃SO₃H·1,5 H₂O (707,773) | | | | | |
|---|---|---|---|---|---|
| Ber. | C 44,12 | H 7,40 | N 11,87 | S 4,53 | Al 3,81 |
| Gef. | C 44,11 | H 7,29 | N 11,65 | S 4,47 | Al 3,70 |

- HPLC (Bedingungen wie im Beispiel 1):: R_{f}-Wert = 7 Minuten,
- Löslichkeiten:: gut löslich in Wasser.

Beispiel 4: Analog Beispiel 3, jedoch ohne Digerieren des Lyophilisats, erhält man aus 6,56 g (10 mMol) Desferrioxamin B-methansulfonat in 100 ml Wasser und 4,94 g (12 mMol) Indium(III)-acetylacetonat in 100 ml Essigsäureethylester weisses, leicht hygroskopisches Indium-oxamin B-methansulfonat.

| C₂₅H₄₅InN₆O₈·CH₃SO₃H (768,593) | | | |
|---|---|---|---|
| Ber. | C 40,63 | H 6,43 | N 10,93 |
| Gef. | C 40,50 | H 6,40 | N 10,90 |

- Massenspektrum [(+) FAB, Thioglycerin]:: (M+H)⁺ = 673.

### HPLC:

Säule: Hypersil ODS, 5µm, 120 x 4,6 mm
Systeme: Lösung A = 2,5 mMol Phosphatpuffer pH 3,0
Lösung B = 20 % Lösung A und 80 % Acetonitril
Gradient:

| Minuten | % A | % B | Fluss: ml/min |
|---|---|---|---|
| 0 | 100 | 0 | 2,3 |
| 10 | 60 | 40 | 2,3 |
| 12 | 0 | 100 | 2,3 |
| 15 | 100 | 0 | 2,3 |

R_{f}-Wert: 9 Minuten,
Löslichkeiten: gut löslich in Wasser und in DMSO.

Beispiel 5: Analog Beispiel 4 erhält man aus 3,28 g (5 mMol) Desferrioxamin B-methansulfonat in 50 ml Wasser und 2,20 g (6 mMol) Gallium(III)-acetylacetonat in 50 ml Essigsäureethylester weisses, leicht hygroskopisches Gallium-oxamin B-methansulfonat.

| C₂₅H₄₅GaN₆O₈·CH₃SO₃H (723,493) | | | |
|---|---|---|---|
| Ber. | C 43,16 | H 6,83 | N 11,62 |
| Gef. | C 43,1 | H 6,8 | N 11,5 |

Massenspektrum [(+) FAB, Thioglycerin]: (M+H)⁺ = 627.
HPLC (Bedingungen wie im Beispiel 4): R_{f}-Wert = 7,3 Minuten,
Löslichkeiten:

| | |
|---|---|
| in Wasser | 30%ig, |
| in DMSO | 20%ig, |
| in Polyethylenglykol 400 | 2%ig. |

Beispiel 6: Eine Suspension von 5,26 g (10 mMol) Desferrioxamin E in 500 ml Wasser wird mit einer Lösung von 5,29 g (15 mMol) Eisen(III)-acetylacetonat in 300 ml Essigsäureethylester versetzt und 5 Stunden stark bei Raumtemperatur gerührt. Anschliessend wird die wässrige Phase mehrmals mit Essigsäureethylester extrahiert und dann lyophilisiert, worauf man Ferrioxamin E erhält.
- HPLC (Bedingungen wie in Beispiel 11):: R_{f}-Wert = 3,92 Minuten (Edukt: 4,70 Minuten).

| C₂₇H₄₅FeN₆O₉·2,5 H₂O (698,58) | | | | |
|---|---|---|---|---|
| Ber. | C 46,42 | H 7,21 | Fe 7,99 | N 12,03 |
| Gef. | C 46,35 | H 7,15 | Fe 8,02 | N 11,77 |

- Massenspektrum [(+) FAB in Thioglycerin]:: (M+H)⁺ = 654.
- Löslichkeit:: in Wasser 30%ig, in DMSO 20%ig, in Polyethylenglykol 400 2%ig.

Beispiel 7: Analog Beispiel 6 erhält man aus einer Suspension von 4,76 g (20 mMol) Desferrithiocin (freie Säure) in 200 ml Wasser und 7,05 g (20 mMol) Eisen(III)-acetylacetonat in 300 ml Essigsäureethylester nach 60minütigem Rühren Ferrithiocin.

R_{f} = 0,50 (Methylenchlorid:Methanol:Wasser = 130:50:8),
- zum Vergleich:: R_{f} von Desferrithiocin = 0,40,
- Löslichkeit:: gut löslich in Wasser.

Beispiel 8: Analog Beispiel 7 erhält man aus einer Suspension von 4,76 g (20 mMol) Desferrithiocin (freie Säure) in 200 ml Wasser und 7,00 g (20 mMol) Mangan(III)-acetylacetonat in 300 ml Essigsäureethylester grünes Manganthiocin.

R_{f} = 0,45 (Methylenchlorid:Methanol:Wasser = 130:50:8)
- Zum Vergleich:: R_{f} von Desferrithiocin = 0,40.

Beispiel 9: Eine Suspension von 9,52 g (40 mMol) Desferrithiocin und 10,41 g (40 mMol) Desferrithiocin-natriumsalz in 400 ml Wasser wird mit 14,10 g (40 mMol) Eisen(III)-acetylacetonat in 400 ml Diethylether versetzt und 1 Stunde stark bei Raumtemperatur gerührt. Dann wird die rotgefärbte wässerige Phase mehrmals mit Diethylether extrahiert und anschliessend lyophilisiert, worauf man Ferrithiocin-natriumsalz erhält.

| C₂₀H₁₆FeN₄NaO₆S₂·2H₂O (587,369) | | | | |
|---|---|---|---|---|
| Ber. | C 40,90 | H 3,43 | N 9,54 | S 10,92 |
| Gef. | C 41,12 | H 3,47 | N 9,66 | S 11,15 |

Beispiel 10: Eine Suspension von 3,78 g (30 mMol) 3-Hydroxy-2-methyl-4-pyron (Maltol) in 500 ml Wasser wird mit 5,29 g (15 mMol) Eisen(III)-acetylacetonat in 500 ml Essigsäureethylester versetzt und 3 Stunden bei Raumtemperatur stark gerührt. Anschliessend wird die wässrige Phase mehrmals mit Essigsäureethylester extrahiert und dann lyophilisiert, wobei man den Eisen(III)-Maltol-Komplex erhält.

| C₁₈H₁₅FeO₉ (431,163) | | | | |
|---|---|---|---|---|
| Ber. | C 49,96 | H 3,57 | Fe 12,91 | H₂O 0,37 |
| Gef. | C 49,77 | H 3,64 | Fe 13,10 | H₂O 0,37 |

HPLC (Bedingungen wie im Beispiel 1, jedoch Gradient nach 14 Minuten 100 % A und 0 % B): 4,85 Minuten (Edukt: 3,75 Minuten),
- Löslichkeiten:: 10%ig in DMSO, 3%ig in Wasser.

Beispiel 11: Eine Suspension von 1,5 g (7,5 mMol) L-Mimosin ((L)-2-Amino-3-[3-hydroxy-pyrid-4-on-1-yl]-propionsäure, vgl. The Merck Index, 10. Auflage, Referat 6065) in 500 ml Wasser wird mit 3,2 g (9 mMol) Eisen(III)-acetylacetonat in 200 ml Essigsäureethylester versetzt und 3 Stunden bei Raumtemperatur stark gerührt. Dann wird die wässerige Phase mehrmals mit insgesamt 2000 ml Essigsäureethylester extrahiert und lyophilisiert, worauf man den Eisen(III)-Mimosin-Komplex erhält.

| C₂₄H₂₇FeN₆O₁₂·2H₂O (683,393) | | | |
|---|---|---|---|
| Ber. | C 42,18 | H 4,57 | N 12,30 |
| Gef. | C 41,95 | H 4,56 | N 12,00 |

HPLC (abgesehen von nachstehend angegebenen Gradienten Bedingungen wie im Beispiel 1):
Gradient:

| Minuten | % A | % B | Fluss: ml/min |
|---|---|---|---|
| 0 | 100 | 0 | 2,3 |
| 12 | 0 | 100 | 2,3 |
| 14 | 100 | 0 | 2,3 |
| 15 | 100 | 0 | 2,3 |

R_{f}-Wert: 0,54 Minuten (Edukt: 0,62 Minuten)
Löslichkeit: ca. 5%ig in Wasser

Beispiel 12: Eine Suspension von 4,17g (30 mMol) 3-Hydroxy-1,2-dimethyl-4-pyridon (beschrieben im Europäischen Patent Nr. 93498, Beispiel 3) in 300 ml Wasser wird mit 5,29 g (15 mMol) Eisen(III)-acetylacetonat in 300 ml Essigsäureethylester versetzt und 3 Stunden lang bei Raumtemperatur intensiv gerührt. Dann wird die wässrige Phase mehrmals mit Essigsäureethylester extrahiert und lyophilisiert. Das Lyophilisat wird zur weiteren Reinigung in 300 ml Essigsäureethylester digeriert, worauf man den Eisen(III)-3-Hydroxy-1,2-dimethyl-4-pyridon-Komplex erhält.

| C₂₁H₂₄FeN₃O₆·1,3 H₂O (493,709) | | | |
|---|---|---|---|
| Ber. | C 51,09 | H 5,43 | N 8,51 |
| Gef. | C 51,03 | H 5,38 | N 8,34 |

- HPLC (Bedingungen wie im Beispiel 11):: R_{f} = 4,10 Minuten (Edukt: 13,63 Minuten).
- Löslichkeit:: 10%ig in DMSO, 20%ig in Wasser.

Beispiel 13: Zu 2,0 g (10 mMol) 3-Hydroxy-2-methyl-N-propyl-4-pyridon-hydrochlorid (beschrieben im Europäischen Patent Nr. 93498, Beispiel 4) in 40 ml Wasser gibt man 4,3 g (12 mMol) Eisen(III)-acetylacetonat in 40 ml Essigsäureethylester und rührt 3 Stunden intensiv bei Raumtemperatur. Dann wird die wässerige Phase mehrmals mit insgesamt 3000 ml Essigsäureethylester extrahiert und lyophilisiert, worauf man den Eisen(III)-3-Hydroxy-2-methyl-N-propyl-4-pyridon-hydrochlorid-Komplex erhält.
- HPLC (Bedingungen wie in Beispiel 11):: R_{f} = 6,51 Minuten (Edukt: 6,33 Minuten).
- Löslichkeit:: 10%ig in DMSO, 20%ig in Wasser.

Beispiel 14: Analog Beispiel 12 setzt man 3,44 g (10 mMol) Rhodotorulasäure (käuflich bei Sigma Chem. Company, P.O. Box 14508, St. Louis M.O. 63178, USA) in 500 ml Wasser mit 3,53 g (10 mMol) Eisen(III)-acetylacetonat in 500 ml Essigsäureethylester um. Nach Aufarbeitung analog Beispiel 12 erhält man den Eisen(III)-Rhodotorulasäure-Komplex.

| C₄₂H₆₆Fe₂N₁₂O₁₈·1 H₂O (1156,77) | | | |
|---|---|---|---|
| Ber. | C 43,61 | H 5,93 | N 14,53 |
| Gef. | C 43,68 | H 5,84 | N 14,41 |

- HPLC (Bedingungen wie in Beispiel 11):: R_{f} = 1,22 Minuten (Edukt: 3,22 Minuten).
- Löslichkeit:: 10%ig in DMSO, 5%ig in Wasser.

## Patentansprüche

1. Verfahren zur Herstellung eines Komplexes aus einem Metallion und einem Chelatbildner, dadurch gekennzeichnet, dass man einen Komplex aus einer β-Dicarbonylverbindung und dem genannten Metallion, welcher in einem mit Wasser nicht beliebig mischbaren organischen Lösungsmittel gut löslich ist, mit einer stöchiometrischen Menge oder einem Unterschuss eines Chelatbildners, dessen Bindungsaffinität zum Metallion höher als diejenige der β-Dicarbonylverbindung ist, oder eines Salzes eines solchen Chelatbildners mit mindestens einer salzbildenden Gruppe umkomplexiert.

2. Verfahren nach Anspruch 1, worin das Metallion ein paramagnetisches Metallion aus der Reihe der Uebergangsmetalle einschliesslich der Lanthaniden ist.

3. Verfahren nach Anspruch 1, worin das Metallion ein Eisen(III)-ion ist.

4. Verfahren nach Anspruch 1, worin das Metallion ein Radionuklidion ist.

5. Verfahren nach einem der Ansprüche 1-4, worin der Chelatbildner Desferrioxamin B oder ein Säureadditionssalz davon ist.

6. Verfahren nach einem der Ansprüche 1-4, worin der Chelatbildner Desferrioxamin E oder ein Salz davon ist.

7. Verfahren nach einem der Ansprüche 1-4, worin der Chelatbildner Desferrithiocin oder ein Salz davon ist.

8. Verfahren nach einem der Ansprüche 1-7, worin man ein Acetylacetonat des Metallions mit dem Chelatbildner umsetzt.

9. Verfahren nach einem der Ansprüche 1-8, dadurch gekennzeichnet, dass man die Reaktion in einem System aus Wasser und einem mit Wasser nicht oder nur wenig mischbaren organischen Lösungsmittel unter intensivem Rühren bei einer Temperatur zwischen +15°C und +40°C durchführt.

10. Verfahren nach Anspruch 9, worin das organische Lösungsmittel Essigsäureethylester oder Diethylether ist.

11. Verfahren nach Anspruch 9 oder 10, dadurch gekennzeichnet, dass man zur Isolierung des gewünschten Metallkomplexes aus dem Reaktionsgemisch zunächst die wässerige Phase abtrennt und mit einem organischen Lösungsmittel extrahiert, in dem der gewünschte Metallkomplex möglichst schlecht und die Verunreinigungen möglichst gut löslich sind, und dann die wässerige Phase lyophilisiert.

## Claims

1. A process for the preparation of a complex of a metal ion and a chelating agent, which comprises transcomplexing a complex of a β-dicarbonyl compound and the said metal ion, which complex is readily soluble in an organic solvent that is not miscible in all proportions with water, with a stoichiometric amount or a less than equivalent amount of a chelating agent whose binding affinity for the metal ion is greater than that of the β-dicarbonyl compound, or of a salt of such a chelating agent containing at least one salt-forming group.

2. A process according to claim 1, wherein the metal ion is a paramagnetic metal ion selected from the series of the transition metals, including the lanthanides.

3. A process according to claim 1, wherein the metal ion is an iron(III) ion.

4. A process according to claim 1, wherein the metal ion is a radionuclide ion.

5. A process according to any one of claims 1 to 4, wherein the chelating agent is desferrioxamine B or an acid addition salt thereof.

6. A process according to any one of claims 1 to 4, wherein the chelating agent is desferrioxamine E or a salt thereof.

7. A process according to any one of claims 1 to 4, wherein the chelating agent is desferrithiocine or a salt thereof.

8. A process according to any one of claims 1 to 7, wherein an acetylacetonate of the metal ion is reacted with the chelating agent.

9. A process according to any one of claims 1 to 8, wherein the reaction is carried out in a system consisting of water and a water-immiscible or substantially water-immiscible organic solvent, with efficient stirring, in the temperature range from +15 to 40°C.

10. A process according to claim 9, wherein the organic solvent is ethyl acetate or diethyl ether.

11. A process according to either claim 9 or claim 10, wherein the desired metal complex is isolated from the reaction mixture by first separating the aqueous phase and extracting it with an organic solvent in which the desired metal complex has as low a solubility as possible and in which the impurities are as readily soluble as possible, and then lyophilising the aqueous phase.

## Revendications

1. Procédé pour la préparation d'un complexe formé d'un ion métallique et d'un chélateur, caractérisé en ce que l'on transforme un complexe formé d'un composé β-dicarbonylé et dudit ion métallique, facilement soluble dans un solvant organique qui n'est que partiellement miscible à l'eau, avec une quantité stoechiométrique ou une quantité inférieure à la quantité stoechiométrique d'un chélateur, dont l'affinité de liaison pour l'ion métallique est plus élevée que celle du composé β-dicarbonylé ou d'un sel d'un tel chélateur comportant au moins un groupe salifiable.

2. Procédé selon la revendication 1 dans lequel l'ion métallique est un ion métallique paramagnétique appartenant à la série des métaux de transition incluant les lanthanides.

3. Procédé selon la revendication 1 dans lequel l'ion métallique est l'ion fer (III).

4. Procédé selon la revendication 1 dans lequel l'ion métallique est un ion radionucléide.

5. Procédé selon l'une des revendications 1 à 4 dans lequel le chélateur est la desferrioxamine B ou l'un de ses sels d'addition avec les acides.

6. Procédé selon l'une des revendications 1 à 4 dans lequel le chélateur est la desferrioxamine E ou l'un de ses sels.

7. Procédé selon l'une des revendications 1 à 4 dans lequel le chélateur est la desferrithiocine ou l'un de ses sels.

8. Procédé selon l'une des revendications 1 à 7 dans lequel on fait réagir un acétylacétonate de l'ion métallique avec le chélateur.

9. Procédé selon l'une des revendications 1 à 8, caractérisé en ce que l'on effectue la réaction dans un système formé d'eau et d'un solvant organique non miscible ou peu miscible à l'eau, en agitant de façon intensive, à une température comprise entre +15°C et +40°C.

10. Procédé selon la revendication 9 dans lequel le solvant organique est l'acétate d'éthyle ou le diéthyléther.

11. Procédé selon la revendication 9 ou 10, caractérisé en ce que, pour isoler le complexe métallique désiré du mélange réactionnel, on sépare tout d'abord la phase aqueuse, puis on extrait avec un solvant organique dans lequel le complexe métallique désiré est aussi peu soluble que possible et les impuretés aussi facilement solubles que possible, puis on lyophilise la phase aqueuse.
